**Europäisches Patentamt**

**European Patent Office**

**Office européen des brevets**

(19)

(11) Numéro de publication: **0 134 161**
**B1**

(12) # FASCICULE DE BREVET EUROPEEN

(45) Date de publication du fascicule du brevet:
**24.06.87**

(21) Numéro de dépôt: **84400857.3**

(22) Date de dépôt: **27.04.84**

(51) Int. Cl.⁴: **C 07 F 5/00,** C 07 C 29/70,
C 07 C 31/28

(54) Procédé de préparation d'alcoolates cériques.

(30) Priorité: **09.08.83 US 521787**

(43) Date de publication de la demande:
**13.03.85 Bulletin 85/11**

(45) Mention de la délivrance du brevet:
**24.06.87 Bulletin 87/26**

(84) Etats contractants désignés:
**AT BE CH DE FR GB IT LI NL SE**

(56) Documents cités:
**US - A - 2 922 801**
**US - A - 3 278 571**

(73) Titulaire: **RHONE-POULENC SPECIALITES CHIMIQUES,**
**"Les Miroirs" 18, Avenue d'Alsace, F-92400 Courbevoie**
**(FR)**

(72) Inventeur: **Gradeff, Peter S., P.O. Box 278, Pottersville**
**New Jersey 08979 (US)**
Inventeur: **Schreiber, Fred G., 100 Lawrence Avenue,**
**Highland Park New Jersey 08904 (US)**

(74) Mandataire: **Dutruc-Rosset, Marie-Claude et al,**
**RHONE-POULENC INTERSERVICES Service Brevets**
**Chimie 25, Quai Paul Doumer, F-92408 Courbevoie**
**Cédex (FR)**

**Description**

La présente invention a pour objet un nouveau procédé de préparation d'alcoolates cériques. Elle concerne également l'obtention d'alcoolates cériques dérivés d'alcools supérieurs à partir d'alcoolates cériques dérivés d'alcools inférieurs.

Les alcoolates de métaux polyvalents constituent une classe importante de composés organométalliques à multiples fonctions qui sont très utilisés dans l'industrie. Dans certains cas, leurs utilisations sont parallèles à celles des carboxylates de métaux et d'autres composés organométalliques, mais, par rapport à ces composés, ils présentent des avantages en raison de leurs propriétés catalytiques, leur facilité d'hydrolyse, leur solubilité dans les solvants organiques et leur volatilité. Ils ont été utilisés comme additifs pour peintures, agents hydrofuges, promoteurs d'adhérence, mordants, agents liants dans les compositions d'émaillage, catalyseurs et, ce qui est aussi très important, comme intermédiaires dans la synthèse d'autres composés organiques.

Il existe quatre méthodes générales de préparation des alcoolates métalliques, toutes mises en oeuvre dans des conditions anhydres, à savoir:

A. Réaction de l'alcool correspondant avec le métal tel que métaux alcalins, métaux alcalinoterreux et aluminium en présence d'un catalyseur alcalin ou acide.

B. Réaction de l'alcool correspondant avec les oxydes et hydroxydes métalliques tels que par exemple NaOH ou $Na_2O$, $V_2O_5$ et $MoO_3$, $2H_2O$.

C. Réaction de l'alcool correspondant avec l'halogénure métallique en présence d'une base anhydre. L'exemple typique est fourni par la préparation de $Th(OR)_4$ ou de $Zr(OR)_4$:

$ThCl_4 + 4ROH + 4NaOR \rightarrow Th(OR)_4 + 4NaCl$

$ZrCl_4 + 4ROH + 4NH_3 \rightarrow Zr(OR)_4 + NH_4Cl$

On peut mettre en oeuvre cette réaction pour préparer des alcoolates de titane, d'hafnium, de germanium, de niobium, de tantale, d'aluminium et d'étain.

D. Transestérification des alcoolates métalliques d'alcools inférieurs tels que méthylates, éthylates ou isopropylates avec un alcool supérieur.

L.Brown et K.Mazdiyasni [Inorganic Chemistry (1970), 2783] ont préconisé la méthode A pour la préparation d'un certain nombre d'alcoolates d'yttrium, de lanthane et d'autres lanthanides. Auparavant, on pensait que la réaction ne pouvait être mise en oeuvre que pour les métaux alcalins, le magnésium et l'aluminium; L.Brown et al l'ont étendue à la synthèse de l'yttrium et de tous les isopropylates de lanthanides. Pour les lanthanides inférieurs tels que le lanthane, le cérium, le praséodyme et le néodyme, on utilise comme catalyseur un mélange de $HgCl_2$ et de $Hg(C_2H_3O_2)_2$ ou de $HgI_2$ pour augmenter à la fois la vitesse de réaction et son rendement. En général, on fait réagir 5 g de tournure de métal avec environ 300 cm$^3$ d'alcool isopropylique à la température de reflux pendant environ 24 heures, en présence d'une petite quantité de catalyseur à base de sel de mercure. Les rendements seraient de 75% ou plus.

La plupart des autres exemples que l'on trouve dans la litérature consacrée à la préparation des alcoolates de lanthanides se réfèrent à la mise en oeuvre des halogénures métalliques correspondants. Dans certains cas, on préfère utiliser à la place de $LaCl_3$, un complexe $LaCl_3$ 3ROH [Misra et al, Austr. J. Chem. 21 797 (1978) et Mehrotra et Batwara, Inorganic Chem. 9 2505 (1970)].

Tripathi, Batwara et Mehrotra [J.C.S.A. (1967) 991] ont proposé une variante intéressante de la méthode D. Ils ont synthétisé des alcoolates d'ytterbium inférieurs (tels que le méthylate et l'éthylate) à partir d'isopropylate d'ytterbium par transestérification avec du méthanol ou de l'éthanol. En raison de leur faible solubilité, les alcools en question ont été éliminés par précipitation durant la réaction, permettant ainsi de parachever la transestérification.

En général, les méthodes A, B et C conviennent uniquement à la préparation d'alcoolates inférieurs tels que méthylates, éthylates et isopropylates étant donné que la réactivité des alcools supérieurs diminue lorsque leur poids moléculaire augmente. Il est préférable de préparer les alcoolates supérieurs à l'aide de la méthode D qui est un procédé en deux étapes.

La seule méthode connue de préparation d'alcoolates cériques appliquait la méthode C au chlorure cérique. Bradley et al [J.C.S. (1956) 2260–64]. Mais comme le tétrachlorure de cérium est instable, Bradley et al. avaient choisi le complexe d'hexachlorocérate (IV) de dipyridinium comme produit de départ.

Dans un premier temps, le dioxyde de cérium est transformé en sulfate cérique d'ammonium. On précipite l'hydroxyde cérique pur à partir d'une solution aqueuse de sulfate cérique d'ammonium et on le lave à fond. On traite l'hydroxyde cérique, fraîchement préparé, en suspension dans de l'alcool absolu avec de l'acide chlorhydrique anhydre et l'on ajoute ensuite de la pyridine pour obtenir le complexe insoluble d'hexachlorocérate (IV) de dipyridinium $(Py)_2CeCl_6$. On filtre et sèche le complexe et on l'utilise pour préparer directement le méthylate, l'éthylate et l'isopropylate; les alcoolates de propyle, butyle, sec-butyle, néopentyle et de n-pentyle étant obtenus par transalcoolyse, c'est-à-dire par transestérification, à partir de l'isopropylate. Le méthylate et l'éthylate sont aussi obtenus par transalcoolyse à partir de l'isopropylate.

La présente invention a pour objet un nouveau procédé de préparation d'alcoolates cériques caractérisé par le fait qu'il consiste à faire réagir l'hexanitratocérate (IV) d'ammonium appelé plus communément nitrate céri-ammoniacal avec un alcool, dans des conditions anhydres, en présence d'une base anhydre, à une température comprise entre −30 °C et 200 °C, jusqu'à formation de l'alcoolate cérique et du sel nitrate de la base.

Ce procédé évite d'avoir dans un premier temps, à préparer, comme l'ont décrit Bradley et al. l'hydroxyde cérique à partir du sel cérique, dans leur cas à partir du sulfate cérique d'ammonium, et à convertir ensuite l'hydroxyde en chlorure qui doit être stabilisé sous forme d'un complexe pyridinium. Le procédé de l'invention est direct, économique et, de plus, il utilise un nitrate céri-ammoniacal que l'on trouve dans le commerce et qui est relativement bon marché.

On pense que les alcoolates de cérium existent sous la forme de l'alcoolate et de complexes par association avec l'alcool libre et, comme ces derniers se révèlent instables, sous la forme de leurs produits de décomposition. Les mélanges de tous ces produits constituent ce que l'on appelle communément «alcoolate de cérium». C'est dans ce sens, communément accepté, que l'on utilise ce terme ici.

Le procédé de l'invention est mis en oeuvre aisément avec les alcools aliphatiques inférieurs ayant de 1 à 5 atomes de carbone, tels que par exemple le méthanol, l'éthanol, le propanol, l'alcool isopropylique, le butanol, l'alcool isobutylique, l'alcool sec-butylique, l'alcool tert-butylique, le pentanol, l'alcool isopentylique, l'alcool sec-pentylique et l'alcool tert-pentylique. Si l'on souhaite obtenir un alcoolate cérique d'un alcool aliphatique supérieur ayant au moins 6 et jusqu'à 20 atomes de carbone, on peut faire appel par exemple, à l'hexanol, l'heptanol, l'alcool isoheptylique, l'octanol, l'alcool isooctylique, l'éthyl-2 hexanol, l'alcool sec-octylique, l'alcool tert-octylique, le nonanol, l'alcool isononylique, le décanol, le dodécanol, le tétradécanol, l'octadécanol, l'hexadécanol, l'alcool oléylique et l'alcool eicosylique. On peut mettre en oeuvre aussi un alcool cycloaliphatique ayant de 3 à 20 atomes de carbone, tel que par exemple le cyclopropanol, le cyclobutanol, le cyclopentanol, le cyclohexanol, le cycloheptanol, le cyclooctanol, le cyclododécanol, le tripropylcyclohexanol, le méthylcyclohexanol et le méthylcycloheptanol; ou bien un alcool aliphatique porteur d'un groupement aromatique ayant de 7 à 20 atomes de carbone tel que par exemple l'alcool benzylique, l'alcool phénéthylique, l'alcool phénylpropylique, l'alcool phényloctadécylique et l'alcool naphtyldécylique.

Un autre objet de l'invention est un procédé de préparation d'alcoolates cériques d'alcools supérieurs à partir d'alcoolates cériques d'alcools inférieurs caractérisé par le fait qu'il consiste à effectuer le transestérification de l'acoolate cérique de l'alcool inférieur, avec l'alcool supérieur, dans des conditions anhydres, en présence d'une base anhydre, à une température comprise entre −30 °C et 200 °C.

Selon la présente invention, la transestérification peut avoir lieu soit simultanément, soit consécutivement à la préparation de l'alcoolate cérique de l'alcool inférieur.

On peut introduire l'alcool supérieur directement dans le mélange réactionnel en même temps qu'un alcool aliphatique inférieur ayant de 1 à 5 atomes de carbone. Le produit réactionnel final que l'on obtient est l'alcoolate cérique de l'alcool supérieur, mais on pense que l'alcool inférieur accélère la réaction en formant dans un premier temps, un alcoolate avec le cérium puis ledit alcoolate étant converti par transestérification avec l'alcool supérieur en un alcoolate de l'alcool supérieur.

On facilite cette conversion si, au cours de la réaction on élimine du mélange réactionnel par distillation, l'alcool aliphatique inférieur libre formé par déplacement dans la réaction de transestérification. Cette opération permet de parachever la réaction de transestérification. L'alcoolate supérieur selon la présente invention est donc obtenu en une seule étape par toute une série de réactions qui se déroulent simultanément dans le même mélange réactionnel sans qu'il soit nécessaire de procéder à la séparation d'un produit réactionnel intermédiaire à savoir un alcoolate inférieur. La seule condition exigée est que l'alcool mis en oeuvre soit stable à l'oxydation de l'ion cérique au cours de la réaction.

On peut effectuer les réactions décrites ci-dessus en présence d'un excès d'alcool qui, s'il s'agit d'un alcool supérieur, peut être un solvant de l'alcoolate correspondant. On peut, également, en plus de l'alcool qui participe à la réaction, utiliser d'autres solvants inertes tels que le benzène, l'hexane et l'acétonitrile. Si on le désire, on peut séparer facilement le solvant du produit réactionnel par distillation sous pression atmosphérique ou réduite lorsque la réaction est terminée.

La réaction se déroule dans des conditions anhydres à une température non critique qui peut varier dans de larges limites entre −30 °C et 200 °C, de préférence entre 0 °C et 150 °C, et encore plus préférentiellement à la température ambiante, température dépendant du système de solvant et de la base employés.

La réaction se déroule en présence d'une base anhydre appropriée telle que l'ammoniac ou un alcoolate de métal alcalin dont l'anion est dérivé, de préférence, du même alcool que celui intervenant dans l'alcoolate cérique recherché afin d'éviter sa contamination par un autre alcoolate. La réaction fournit un produit secondaire qui est le sel nitrate correspondant d'ammonium ou de métal alcalin correspondant.

Comme exemples de bases anhydres, on peut citer le méthylate de sodium, l'éthylate de sodium, le méthylate de potassium, l'éthylate de potassium, l'isopropylate de sodium, l'isobutylate de sodium, le méthylate de lithium et l'éthylate de lithium.

La durée de la réaction n'est pas critique. On poursuit la réaction jusqu'à formation de l'alcoolate désiré. La réaction peut durer de 10 minutes à plusieurs heures, mais il n'est pas nécessaire de poursuivre la réaction au-delà de cinq heures. Habituellement, il faut de une à trois heures pour que la réaction soit terminée.

La réaction peut se dérouler très rapidement à température ambiante et si c'est le cas, il est très vraisemblable qu'elle aura lieu aussi à des températures bien en-dessous de la température ambiante et ce, jusqu'à −30 °C, mais il n'y a aucune raison d'engager des frais supplémentaires pour refroidir le mélange réactionnel. La limite supérieure de la température réactionnelle est imposée par la volatilité du mélange réactionnel ou de l'un de ses composants, et leur température de décomposition. Il n'y a aucune raison de mettre en oeuvre une température supérieure au point d'ébullition du mélange réactionnel à la pression atmosphérique, mais si la température d'ébullition est trop basse comme dans le cas du méthanol par exemple, on peut utiliser un réacteur fermé ou un appareillage sous pression (par exemple de 2 à 3 atmosphères = 2,026·10⁵ à 3,039·10⁵ Pa). Les facteurs ci-dessus étant pris en considération, il est inutile que la température réactionnelle dépasse 200 °C.

La quantité de base anhydre est stoechiométrique étant donné que la fonction du cation de la base, ammonium ou métal alcalin, est de fixer le nitrate provenant du nitrate céri-ammoniacal de départ. Il est possible, mais non nécessaire de mettre en oeuvre un excès.

De même, la quantité d'alcool est au moins la quantité stoechiométrique requise pour réagir avec le nitrate céri-ammoniacal, mais on peut également mettre en oeuvre des quantités plus importantes. Il va de soi que l'on utilisera des quantités supérieures aux quantités stoechiométriques lorsque l'alcool jouera aussi le rôle de solvant et ce, en fonction de la dilution nécessaire du mélange réactionnel.

Le mélange réactionnel contient le sel nitrate du cation de la base, qui peut être séparé de l'alcoolate au cours du procédé. Si ce sel est moins soluble dans le mélange réactionnel que l'alcoolate obtenu, on peut le séparer par filtration du produit réactionnel. Il existe une autre possibilité qui consiste à reprendre le mélange réactionnel dans un solvant inerte tel que benzène, toluène ou hexane, de préférence dans un solvant inerte dans lequel l'alcoolate est soluble et le sel nitrate insoluble et ensuite on sépare le sel nitrate par filtration ou centrifugation.

Dans le cas des alcools inférieurs, le produit réactionnel et le sel nitrate sont solides. Dans ce cas naturellement, on ne peut pas séparer les deux par filtration; on peut récupérer l'alcoolate et le séparer du sel nitrate par extraction avec un solvant de l'alcoolate dans lequel le sel nitrate est insoluble, en utilisant par exemple un appareil Soxhlet. Une autre possibilité consiste à utiliser un solvant du sel nitrate dans lequel l'alcoolate cérique est insoluble. Par exemple, le nitrate d'ammonium est tout-à-fait soluble dans le méthanol alors que le tétraméthylate cérique ne l'est pas et, par conséquent, on peut, dans ce cas, séparer le nitrate d'ammonium par extraction avec le méthanol. On peut également séparer le nitrate de sodium de cette manière bien qu'il soit moins soluble dans le méthanol.

En fonction des conditions de réaction et de mise en oeuvre on peut isoler l'alcoolate sous forme d'associations avec une ou plusieurs molécules d'alcool qui, généralement, le rend plus stable à l'hydrolyse. Par ailleurs, on peut isoler l'alcoolate sous une forme partiellement hydrolysée convenant ou appropriée à certaines applications.

Pour certaines applications, on peut utiliser les alcoolates de cérium sous la forme sous laquelle ils existent dans le mélange réactionnel à la fin de la réaction sans avoir à les isoler du mélange réactionnel ou à les séparer des nitrates, ce qui évite des coûts de traitement et de manipulation.

Les exemples qui suivent illustrent un mode de réalisation préférentielle de l'invention mais en aucun cas, il ne limitent la portée de l'invention.

Dans les exemples, les pourcentages sont exprimés en poids.

Exemple 1
Préparation de tétrabenzylate cérique à partir de nitrate céri-ammoniacal, de méthylate de sodium en solution dans du méthanol et d'alcool benzylique en quantité stoechiométrique

On ajoute 30,1 g (0,1810 mole) d'une solution anhydre de méthylate de sodium à 25% dans le méthanol à une solution de 16,50 g (0,0301 mole) de nitrate céri-ammoniacal dans 60,6 g de méthanol maintenue sous agitation et sous argon afin de conserver les conditions anhydres durant la réaction qui est effectuée à température ambiante. Après avoir agité le mélange jaune vif pendant 15 min, on ajoute 13,0 g (0,1202 mole) d'alcool benzylique, puis 41,0 g de toluène.

Au bout d'une heure, on distille le méthanol et le toluène jusqu'à ce que le mélange, devenu trop épais, ne puisse plus être agité. On ajoute du toluène frais pour garder le résidu sous forme de bouillie tout en continuant de chauffer jusqu'à ce que soit atteinte la température d'ébullition de 104°C.

On sépare le nitrate de sodium par filtration et on le lave quatre fois avec du toluène. On obtient 15,3 g de nitrate de sodium (théorie: 15,35 g). On concentre le filtrat marron foncé sous pression réduite jusqu'à ce qu'il n'y ait plus de liquide à distiller.

On obtient 17,9 g (théorie: 17,11 g) de tétrabenzylate cérique, produit marron foncé sous forme d'une huile fluide.

L'analyse des résidus est la suivante:
– cendres: 30,01% (théorie: 30,27%)
– carbone trouvé: 58,81% (théorie: 59,14%)
– hydrogène trouvé: 5,23% (théorie: 4,96%)

Exemple 2
Préparation de tétraméthylate cérique à partir de nitrate céri-ammoniacal, d'ammoniac et de méthanol

On ajoute de l'ammoniac anhydre (2,1 g) à une solution de nitrate céri-ammoniacal (16,46 g) dans du méthanol (102,90 g) en agitant énergiquement pendant plus de 2 h 30 min. On sépare les solides par filtration et on les lave quatre fois avec du méthanol. Après séchage du résidu, on obtient un solide de 6,11 g, jaune vif, qui est le tétraméthylate cérique. On évapore à sec le filtrat jaune et on obtient 15,42 g d'un solide jaune pâle contenant essentiellement du nitrate d'ammonium et un peu de tétraméthylate cérique qui est à l'origine de la couleur jaune. Le bilan matière concernant le cérium est quantitatif.

**Exemple 3**

Préparation du tétraoctylate cérique à partir de l'alcool octylique, du méthylate de sodium et du méthanol

On ajoute 13,1 g d'une solution anhydre de méthylate de sodium à 25% dans le méthanol à une solution de 5,48 g de nitrate céri-ammoniacal dans 20,0 g de méthanol et 5,2 g l'alcool octylique maintenue sous argon afin de conserver les conditions anhydres durant la réaction. Après agitation, on dilue la bouillie jaune vif avec 25,3 g de toluène. On sépare le méthanol par distillation en chauffant jusqu'au point d'ébullition, à savoir 104°C. On filtre le mélange et on lave les solides 5 fois avec 8 g de toluène. Pendant toute une nuit, on sèche le solide sous vide pour obtenir du nitrate d'ammonium essentiellement pur, dont le poids est de 5,0 g (théorie: 5,1 g).

On concentre le filtrat du mélange réactionnel sous pression réduite pour obtenir 6,8 g de tétraoctylate cérique qui se présente sous la forme d'une huile orange foncé (théorie: 6,6 g). L'analyse des cendres donne 26,8% de cendres, carbone 58,4%, hydrogène 10,5% (théorie: respectivement 26,4%, 58,5%, 10,4%).

**Exemple 4**

Préparation de tétraméthylate cérique à partir de nitrate céri-ammoniacal et de méthylate de sodium

On ajoute une solution de méthylate de sodium à 25% dans le méthanol (260,4 g) à une solution de nitrate céri-ammoniacal (109,5 g) dans du méthanol (440,0 g) maintenue sous agitation et sous argon afin de conserver les conditions anhydres durant la réaction. On chauffe le mélange jaune au reflux pendant 3 h. Ensuite on élimine le solvant sous pression réduite et l'on obtient un solide jaune vif. On transfère ce produit dans une cartouche d'extraction et on chauffe au reflux avec du méthanol pendant toute une nuit afin d'éliminer le nitrate de sodium. On sèche ensuite sous pression réduite le résidu qui reste dans la cartouche, à savoir le tétraméthylate cérique et l'on obtient 50,2 g de tétraméthylate cérique jaune vif (théorie: 52,8 g). On concentre à sec la solution de méthanol et l'on obtient 103 g de solide blanc (théorie: 101,8 g), essentiellement du nitrate de sodium. L'analyse élémentaire montre que le produit n'est que légèrement hydrolysé. Cendres: 66,4%, carbone: 17,11%, hydrogène: 4,2% (théorie: respectivement 65,1%, 18,2%, 4,6%).

**Exemple 5**

Préparation de tétra (acétylacétonate) cérique à partir d'un mélange réactionnel de tétraméthylate cérique

On fait absorber de l'ammoniac (3,9 g, 0,229 mole) par une solution de nitrate céri-ammoniacal (29,88 g, 0,0502 mole) dans 93,90 g de méthanol. Après avoir agité la bouillie jaune vif pendant une heure, on ajoute de l'acétylacétone (20,5 g, 0,2048 mole) goutte-à-goutte, pendant 30 min afin d'obtenir une solution rouge-noire. On évapore le mélange réactionnel à sec sous vide et ensuite, on met le solide en suspension dans du chlorure de méthylène (87,8 g). On sépare les sels par filtration sous forme d'aiguilles blanches (22,13 g, 0,06% de cendres) après les avoir lavés. On évapore à sec le filtrat rouge foncé sous pression réduite et l'on obtient 30,09 g de poudre marron foncé de tétra (acétylacétonate) cérique contenant un peu de nitrate d'ammonium résiduel. Après avoir corrigé les valeurs concernant le nitrate d'ammonium, l'analyse élémentaire donne 33,05% de cendres, 45,64% de carbone et 5,3% d'hydrogène (théorie: respectivement 32,08%, 44,77% et 5,26%).

**Exemple 6**

Préparation de tétraoléylate cérique à partir de nitrate céri-ammoniacal dans du méthanol et de l'ammoniac et d'alcool oléylique en léger excès

L'ammoniac (2,8 g, 0,1644 mole) est absorbé en une heure par une solution de nitrate céri-ammoniacal (16,77 g, 0,0300 mole) dans de l'alcool oléylique (38,04 g) et du méthanol (74,77 g). L'excès de méthanol et d'ammoniac est distillé à partir de la bouillie orange terne et l'on obtient une pâte orange épaisse contenant du tétra(oléylate) cérique et du nitrate d'ammonium. On ajoute de l'hexane (90,2 g) à la pâte pour dissoudre le tétra(oléylate) cérique, puis on sépare par filtration le nitrate d'ammonium (14,75 g, théorie: 14,41 g) contenant aussi un peu de tétra(oléylate) cérique et on le lave avec de l'hexane frais. On concentre le filtrat jusqu'à l'obtention d'une huile jaune-orange foncé, 43,87 g (théorie: 42,12 g), analyse, 7,3% de cendres.

**Exemple 7**

Préparation de tétraméthylate cérique à partir de nitrate d'ammonium cérique, d'ammoniac et de méthanol

On ajoute de l'ammoniac anhydre (24,7 g) très lentement, au-dessus de la surface, d'une solution de nitrate céri-ammoniacal (16,45 g) dans du méthanol (300 g) en agitant énergiquement pendant plus de 5 h. On sépare les solides par filtration et on les lave quatre fois avec du méthanol. On sèche le résidu et on obtient 10,1 g de tétraméthylate cérique solide jaune vif. Le méthylate cérique est un complexe par association avec le méthanol en raison de la présence d'une plus grande quantité de méthanol; il présente une plus grande stabilité vis-à-vis de l'hydrolyse que le produit obtenu dans l'exemple 2. L'analyse élémentaire donne 57,2% de cendres, 18,7% de carbone et 5,37% d'hydrogène (théorie: respectivement 65,1%, 18,2% et 4,6%).

On évapore à sec le filtrat du méthanol et l'on obtient la quantité théorique de nitrate d'ammonium, soit 14,28 g.

**Revendications**

1. Procédé de préparation d'alcoolates cériques, caractérisé par le fait qu'il consiste à faire réagir le nitrate céri-ammoniacal avec un alcool, dans des conditions anhydres, en présence d'une base anhydre, à une température comprise entre –30 °C et 200 °C, jusqu'à formation de l'alcoolate cérique et du sel nitrate de la base.

2. Procédé selon la revendication 1, caractérisé par le fait que l'alcool est un alcool aliphatique inférieur ayant de 1 à 5 atomes de carbone.

3. Procédé selon la revendication 1, caractérisé par le fait que l'alcool est un alcool aliphatique supérieur ayant un moins 6 et jusqu'à 20 atomes de carbone.

4. Procédé selon la revendication 1, caractérisé par le fait que l'alcool est un alcool cycloaliphatique ayant de 3 à 20 atomes de carbone.

5. Procédé selon la revendication 1, caractérisé par le fait que l'alcool est un alcool aliphatique porteur d'un groupement aromatique ayant de 7 à 20 atomes de carbone.

6. Procédé selon la revendication 1, caractérisé par le fait que l'alcool mis en oeuvre est tel qu'il soit stable à l'oxydation de l'ion cérique au cours de la réaction et est choisi dans le groupe comprenant les alcools aliphatiques supérieurs ayant au moins 6 et jusqu'à 20 atomes de carbone, les alcools cycloaliphatiques ayant de 3 à 20 atomes de carbone; les alcools aliphatiques porteurs d'un groupement aromatique ayant de 7 à 20 atomes de carbone, et qu'il est introduit directement dans le mélange réactionnel conjointement avec un alcool aliphatique inférieur ayant de 1 à 5 atomes de carbone.

7. Procédé selon la revendication 1, caractérisé par le fait que la base anhydre est l'ammoniac ou un alcoolate de métal alcalin.

8. Procédé selon la revendication 7, caractérisé par le fait que la base anhydre est un alcoolate de métal alcalin dont l'anion est dérivé du même alcool que celui intervenant dans l'alcoolate cérique recherché.

9. Procédé selon la revendication 1, caractérisé par le fait qu'il est conduit dans un solvant inerte dans lequel le nitrate céri-ammoniacal est soluble.

10. Procédé selon la revendication 9, caractérisé par le fait que le solvant est l'alcool s'il s'agit d'un alcool supérieur, dont sera dérivé l'alcoolate cérique recherché.

11. Procédé selon la revendication 1, caractérisé par le fait que la température réactionnelle est comprise entre 0 °C et 150 °C.

12. Procédé selon la revendication 1, caractérisé par le fait que le produit réactionnel, à savoir l'alcoolate cérique, est récupéré et séparé du sel nitrate par extraction avec un solvant de l'alcoolate ou du sel nitrate.

13. Procédé de préparation d'alcoolates cériques d'un alcool supérieur à partir d'alcoolates cériques d'un alcool inférieur obtenu selon l'une des revendications 1 à 12, caractérisé par le fait qu'il consiste à effectuer la transestérification de l'alcoolate cérique de l'alcool inférieur, avec l'alcool supérieur, dans des conditions anhydres, en présence d'une base anhydre, à une température comprise entre −30 °C et 200 °C.

14. Procédé selon la revendication 13, caractérisé par le fait que l'alcoolate cérique de l'alcool inférieur est un alcoolate cérique d'un alcool aliphatique inférieur ayant de 1 à 5 atomes de carbone.

15. Procédé selon la revendication 13, caractérisé par le fait que l'alcool supérieur est choisi dans le groupe comprenant les alcools aliphatiques supérieurs ayant au moins 6 et jusqu'à 20 atomes de carbone, les alcools cycloaliphatiques ayant de 3 à 20 atomes de carbone et les alcools aliphatiques porteurs d'un groupement aromatique ayant de 7 à 20 atomes de carbone.

16. Procédé selon la revendication 13, caractérisé par le fait que l'on sépare par distillation tout alcool libre se formant au cours de la réaction.

17. Procédé selon la revendication 13, caractérisé par le fait que l'on obtient l'alcoolate cérique de l'alcool supérieur en une seule étape sans qu'il soit nécessaire de procéder à la séparation de l'alcoolate cérique de l'alcool inférieur.

**Patentansprüche**

1. Verfahren zur Herstellung von Cer(IV)-Alkoholaten, dadurch gekennzeichnet, dass es darin besteht, dass man Cer(IV)-Ammoniumnitrat unter wasserfreier Bedingung in Gegenwart einer wasserfreien Base mit einem Alkohol reagieren lässt, bei einer Temperatur zwischen −30 °C und 200 °C, bis zur Bildung von Cer(IV)-Alkoholat und dem Nitratsalz der Base.

2. Verfahren nach Anspruch 1, dadurch gekennzeichnet, dass der Alkohol ein niederer aliphatischer Alkohol ist mit 1 bis 5 Kohlenstoffatomen.

3. Verfahren nach Anspruch 1, dadurch gekennzeichnet, dass der Alkohol ein höherer aliphatischer Alkohol mit mindestens 6 bis zu 20 Kohlenstoffatomen ist.

4. Verfahren nach Anspruch 1, dadurch gekennzeichnet, dass der Alkohol ein cycloaliphatischer Alkohol mit 3 bis 20 Kohlenstoffatomen ist.

5. Verfahren nach Anspruch 1, dadurch gekennzeichnet, dass der Alkohol ein aliphatischer Alkohol ist, der eine aromatische Gruppe trägt mit 7 bis 20 Kohlenstoffatomen.

6. Verfahren nach Anspruch 1, dadurch gekennzeichnet, dass der eingesetzte Alkohol gegenüber Oxydation durch das Cer(IV)-Ion im Verlauf der Reaktion stabil ist und ausgewählt wird aus der Gruppe, umfassend die aliphatischen höheren Alkohole mit 6 bis 20 Kohlenstoffatomen, die cycloaliphatischen Alkohole mit 3 bis 20 Kohlenstoffatomen, die aliphatischen Alkohole, die eine aromatische Gruppe tragen mit 7 bis 20 Kohlenstoffatomen, und dass dieser direkt in die Reaktionsmischung eingeführt wird, gemeinsam mit einem niederen aliphatischen Alkohol, der 1 bis 5 Kohlenstoffatome hat.

7. Verfahren nach Anspruch 1, dadurch gekennzeichnet, dass die wasserfreie Base Ammoniak oder ein Alkoholat eines Alkalimetalls ist.

8. Verfahren nach Anspruch 7, dadurch gekennzeichnet, dass die wasserfreie Base ein Alkoholat eines Alkalimetalls ist, dessen Anion von jenem Alkohol stammt, der in das herzustellende Cer(IV)-Alkoholat eingeführt werden soll.

9. Verfahren nach Anspruch 1, dadurch gekennzeichnet, dass es in einem inerten Solvens, in dem das Cer(IV)-Ammoniumnitrat löslich ist, ausgeführt wird.

10. Verfahren nach Anspruch 9, dadurch gekennzeichnet, dass das Solvens ein Alkohol ist, wenn es sich um einen höheren Alkohol handelt, von dem sich das herzustellende Cer(IV)-Alkoholat ableitet.

11. Verfahren nach Anspruch 1, dadurch gekennzeichnet, dass die Reaktionstemperatur zwischen 0 und 120 °C liegt.

12. Verfahren nach Anspruch 1, dadurch gekennzeichnet, dass das Reaktionsprodukt, nämlich das Cer(IV)-Alkoholat gewonnen und von dem Nitratsalz separiert wird durch Extraktion mit einem Lösungsmittel für das Alkoholat oder für das Nitratsalz.

13. Verfahren zur Herstellung von Cer(IV)-Alkoholaten höherer Alkohole, ausgehend von Cer(IV)-Alkoholaten niederer Alkohole, die nach einem der Ansprüche 1 bis 12 erhalten werden, dadurch gekennzeichnet, dass es darin besteht, dass eine Umesterung des Cer(IV)-Alkoholats des niederen Alkohols mit dem höheren Alkohol bewirkt wird, unter wasserfreien Bedingungen, in Gegenwart einer wasserfreien Base, bei einer Temperatur zwischen −30 °C und 200 °C.

14. Verfahren nach Anspruch 13, dadurch gekennzeichnet, dass das Cer(IV)-Alkoholat des niederen Alkohols ein Cer(IV)-Alkoholat eines niederen aliphatischen Alkohols mit 1 bis 5 Kohlenstoffatomen ist.

15. Verfahren nach Anspruch 13, dadurch gekennzeichnet, dass der höhere Alkohol aus der Gruppe, umfassend die höheren aliphatischen Alkohole mit mindestens 6 und bis zu 20 Kohlenstoffatomen, die cycloaliphatischen Alkohole mit von 3 bis 20 Kohlenstoffatomen und die aliphatischen Alkohole, die eine aromatische Gruppe tragen mit 7 bis 20 Kohlenstoffatomen, ausgewählt wird.

16. Verfahren nach Anspruch 13, dadurch gekennzeichnet, dass man den gesamten freien Alkohol, der sich im Verlauf der Reaktion bildet, durch Destillation abtrennt.

17. Verfahren nach Anspruch 13, dadurch gekennzeichnet, dass man das Cer(IV)-Alkoholat des höheren Alkohols in einem einzigen Schritt erhält, ohne dass es notwendig ist, eine Trennung von dem Cer(IV)-Alkoholat des niederen Alkohols vorzunehmen.

**Claims**

1. Process for the preparation of ceric alcoholates, characterized in that it consists in reacting ceric ammonium nitrate with an alcohol, under anhydrous conditions, in the presence of an anhydrous base, at a temperature of between −30 °C and 200 °C, until ceric alcoholate and the nitrate salt of the base are formed.

2. Process according to claim 1, characterized in that the alcohol is a lower aliphatic alcohol containing from 1 to 5 carbon atoms.

3. Process according to claim 1, characterized in that the alcohol is a higher aliphatic alcohol containing at least 6 and up to 20 carbon atoms.

4. Process according to claim 1, characterized in that the alcohol is an alicyclic alcohol containing from 3 to 20 carbon atoms.

5. Process according to claim 1, characterized in that the alcohol is an aliphatic alcohol carrying an aromatic group containing from 7 to 20 carbon atoms.

6. Process according to claim 1, characterized in that the alcohol employed is such that it is stable against oxidation by the ceric ion during the reaction and is chosen from the group comprising higher aliphatic alcohols containing at least 6 and up to 20 carbon atoms, alicyclic alcohols containing from 3 to 20 carbon atoms; aliphatic alcohols carrying an aromatic group containing from 7 to 20 carbon atoms, and in that it is introduced directly into the reaction mixture together with a lower aliphatic alcohol containing from 1 to 5 carbon atoms.

7. Process according to claim 1, characterized in that the anhydrous base is ammonia or an alcoholate of an alkali metal.

8. Process according to claim 7, characterized in that the anhydrous base is an alcoholate of an alkali metal the anion of which is derived from the same alcohol as that involved in the ceric alcoholate sought.

9. Process according to claim 1, characterized in that it is performed in an inert solvent in which the ceric ammonium nitrate is soluble.

10. Process according to claim 9, characterized in that the solvent is the alcohol, in the case of a higher alcohol, from which the ceric alcoholate sought will be derived.

11. Process according to claim 1, characterized in that the reaction temperature is between 0 °C and 150 °C.

12. Process according to claim 1, characterized in that the reaction product, viz. ceric alcoholate, is recovered and separated from the nitrate salt by extraction with a solvent of the alcoholate or of the nitrate salt.

13. Process for the preparation of ceric alcoholates of a higher alcohol from ceric alcoholates of a lower alcohol obtained according to one of claims 1 to 12, characterized in that it consists in carrying out a transesterification of the ceric alcoholate of the lower alcohol, with a higher alcohol, under anhydrous conditions, in the presence of an anhydrous base, at a temperature of between −30 °C and 200 °C.

14. Process according to claim 13, characterized in that the ceric alcoholate of the lower alcohol is a ceric alcoholate of a lower aliphatic alcohol containing from 1 to 5 carbon atoms.

15. Process according to claim 13, characterized in that the higher alcohol is chosen from the group comprising higher aliphatic alcohols containing at least 6 and up to 20 carbon atoms, alicyclic alcohols containing from 3 to 20 carbon atoms and aliphatic alcohols carrying an aromatic group containing from 7 to 20 carbon atoms.

16. Process according to claim 13, characterized in that any free alcohol formed during the reaction is separated by distillation.

17. Process according to claim 13, characterized in that the ceric alcoholate of the higher alcohol is obtained in a single step without the need for carrying out a separation of the ceric alcoholate from the lower alcohol.